(19) Europäisches Patentamt
European Patent Office
Office européen des brevets

(11) Veröffentlichungsnummer: **0 129 177**
**A2**

(12) # EUROPÄISCHE PATENTANMELDUNG

(21) Anmeldenummer: **84106629.3**

(22) Anmeldetag: **09.06.84**

(51) Int. Cl.³: **C 07 C 121/60**
C 07 C 121/75, C 07 C 120/00
C 09 K 3/34, G 02 F 1/13

(30) Priorität: **17.06.83 CH 3334/83**

(43) Veröffentlichungstag der Anmeldung:
**27.12.84 Patentblatt 84/52**

(84) Benannte Vertragsstaaten:
**CH DE FR GB IT LI SE**

(71) Anmelder: **F. HOFFMANN-LA ROCHE & CO.**
**Aktiengesellschaft**

**CH-4002 Basel(CH)**

(72) Erfinder: **Petrzilka, Martin, Dr.**
**Schwarzackerstrasse 54**
**CH-4303 Kaiseraugst(CH)**

(72) Erfinder: **Villiger, Alois, Dr.**
**Im Ettingerhof 5**
**CH-4055 Basel(CH)**

(74) Vertreter: **Zimmermann, Hans, Dr. et al,**
**Grenzacherstrasse 124 Postfach 3255**
**CH-4002 Basel(CH)**

(54) **Flüssigkristalline Benzonitrile.**

(57) Flüssigkristalline Verbindungen der Formel

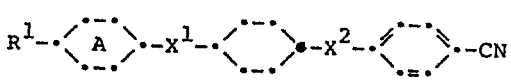

I

worin eine der Gruppen $X^1$ und $X^2$ eine Aethylengruppe $-CH_2CH_2-$ und die andere eine Aethylengruppe $-CH_2CH_2-$ oder eine einfache Kovalenzbindung bezeichnet, Ring A 1, 4-Phenylen oder trans-1, 4-Cyclohexylen darstellt und $R^1$ geradkettiges $C_1$–$C_{12}$-Alkyl oder an einem 1, 4-Phenylenring auch geradkettiges $C_1$–$C_{12}$-Alkoxy bedeutet,

deren Herstellung und Verwendung für elektro-optische Zwecke sowie flüssigkristalline Gemische, die solche Verbindungen enthalten, werden beschrieben.

EP 0 129 177 A2

F.Hoffmann-La Roche & Co.Aktiengesellschaft, Basel/Schweiz

RAN 6220/041

## Flüssigkristalline Benzonitrile

Die vorliegende Erfindung betrifft neue flüssigkristalline Verbindungen und deren Herstellung. Die Erfindung betrifft ebenfalls die Verwendung dieser Verbindungen für elektro-optische und chromatographische Zwecke und flüssigkristalline Gemische, die solche Verbindungen enthalten.

Flüssigkristalline Materialien besitzen vor allem als Dielektrika für elektro-optische Anzeigevorrichtungen Bedeutung, da die optischen Eigenschaften derartiger Materialien durch elektrische Felder beeinflusst werden können. Elektro-optische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Typ), dem Schadt-Helfrich-Effekt (Drehzelle), einem cholesterisch-nematischen Phasenübergang oder dem Gast/Wirt-Effekt ("Guest/Host-Zelle").

Für eine erfolgreiche technische Anwendung dieser Effekte ist es jedoch wichtig, dass die verwendeten Materialien einer Vielzahl von weiteren Anforderungen genügen.

Zim/26.3.84

Beispielsweise müssen sie eine gute chemische Stabilität gegenüber Wärme, Feuchtigkeit, Luft, elektromagnetische Strahlung (im ultravioletten, sichtbaren und infraroten Bereich), elektrischen Feldern und dergleichen besitzen, farblos sein und bei der Anwendung in einer Zelle einen guten Kontrast ergeben. Ferner sollten sie niedere Viskosität und kurze Ansprechzeiten aufweisen und im ganzen Temperaturbereich, in welchem die Flüssigkristallzelle betrieben werden soll, eine nematische bzw. cholesterische Mesophase besitzen. Weitere Eigenschaften, wie beispielsweise die dielektrische Anisotropie, die Schwellenspannung und die Leitfähigkeit, müssen je nach verwendetem Zellentyp unterschiedliche Bedingungen erfüllen.

Da es im allgemeinen nicht möglich ist, alle gewünschten und zum Teil widersprüchlichen Eigenschaften mit einer einzigen Verbindung zu erreichen, werden in der Regel Mischungen von zwei oder mehreren Verbindungen hergestellt. Hierbei ist jedoch zu beachten, dass die Komponenten untereinander keine chemischen Reaktionen eingehen dürfen und auch bei Raumtemperatur und tieferen Temperaturen gut mischbar sein müssen. Ferner sollten die Mischungen mindestens bei Temperaturen, bei denen die Flüssigkristallzelle betrieben werden soll, keine smektischen Mesophasen aufweisen.

Flüssigkristalle mit positiver dielektrischer Anisotropie ($\Delta\varepsilon = \varepsilon_{\parallel} - \varepsilon_{\perp} > 0$, wobei $\varepsilon_{\parallel}$ die Dielektrizitätskonstante entlang der Moleküllängsachse und $\varepsilon_{\perp}$ die Dielektrizitätskonstante senkrecht dazu bedeuten) orientieren sich in einem elektrischen Feld mit der Richtung der grössten Dielektrizitätskonstante, d.h. mit den Moleküllängsachsen parallel zur Feldrichtung. Dieser Effekt wird unter anderem in der erwähnten Drehzelle und Guest/Host-Zelle ausgenützt. Um die Schwellenspannung niedrig zu halten, sollte hierbei das verwendete Flüssigkristallmaterial eine relativ hohe Anisotropie der Dielektrizitätskonstante aufweisen. Andererseits haben aber Verbindungen mit grosser positiver Anisotropie der Dielektrizitätskonstante im allgemeinen den Nachteil, dass sie zugleich die Viskosität des Flüssigkristallmaterials erhöhen.

Gegenstand der vorliegenden Erfindung sind nun die Verbindungen der allgemeinen Formel

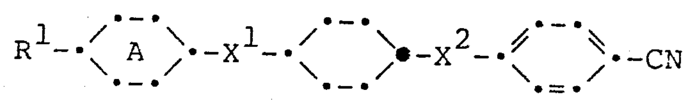

I

worin eine der Gruppen $X^1$ und $X^2$ eine Aethylengruppe $-CH_2CH_2-$ und die andere eine Aethylengruppe $-CH_2CH_2-$ oder eine einfache Kovalenzbindung bezeichnet, Ring A 1,4-Phenylen oder trans-1,4-Cyclohexylen darstellt und $R^1$ geradkettiges $C_1$-$C_{12}$-Alkyl oder an einem 1,4-Phenylenring auch geradkettiges $C_1$-$C_{12}$-Alkoxy bedeutet.

Die erfindungsgemässen Verbindungen besitzen zugleich grosse positive Anisotropien der Dielektrizitätskonstanten und hohe Klärpunkte und trotzdem vergleichsweise niedrige Viskositäten. Sie sind farblos, besitzen eine sehr gute chemische und photochemische Stabilität und weisen auch die übrigen geforderten Eigenschaften auf. Da sie mit andern Flüssigkristallen gut mischbar sind, besitzen sie einen breiten Anwendungsbereich.

Ferner sind die erfindungsgemässen Verbindungen auch für oder als stationäre Phasen in der Gaschromatographie verwendbar. Insbesondere eignen sie sich zur Trennung von Isomerengemischen, z.B. cis/trans-disubstituierten Cyclohexanen, Doppelbindungsisomeren und dergleichen.

Die obige Formel I umfasst p-[trans-4-[2-(p-Alkylphenyl- oder p-Alkoxyphenyl)äthyl]cyclohexyl]benzonitrile, p-[trans-4-[2-(trans-4-Alkylcyclohexyl)äthyl]cyclohexyl]-benzonitrile, p-[2-[trans-4-(p-Alkylphenyl oder p-Alkoxyphenyl)cyclohexyl]äthyl]benzonitrile, p-[2-[trans-4-(trans-4-Alkylcyclohexyl)cyclohexyl]äthyl]benzonitrile, p-[2-[trans-4-[2-(p-Alkylphenyl oder p-Alkoxyphenyl)äthyl]-cyclohexyl]äthyl]benzonitrile und p-[2-[trans-4-[2-(trans-4-Alkylcyclohexyl)äthyl]cyclohexyl]äthyl]benzonitrile, worin Alkyl jeweils Methyl, Aethyl, Propyl, Butyl, Pentyl, Hexyl, Heptyl, Octyl, Nonyl, Decyl, Undecyl und Dodekyl bedeutet und Alkoxy jeweils Methoxy, Aethoxy, Propyloxy, Butyloxy, Pentyloxy, Hexyloxy, Heptyloxy, Octyloxy, Nonyloxy, Decyloxy, Undecyloxy und Dodecyloxy bedeutet.

Bevorzugte Alkyl- und Alkoxyreste $R^1$ sind $C_1$-$C_7$-Alkyl und $C_1$-$C_6$-Alkoxy. Ferner sind grundsätzlich diejenigen Verbindungen der Formel I bevorzugt, worin $R^1$ geradkettiges Alkyl bedeutet.

Die Verbindungen der Formel I können erfindungsgemäss dadurch hergestellt werden, dass man

a) zur Herstellung der Verbindungen der Formel I, worin $X^2$ eine Aethylengruppe bedeutet, eine Verbindung der allgemeinen Formel

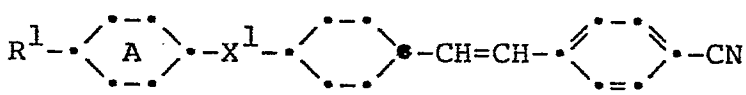

II

worin $X^1$ eine Aethylengruppe $-CH_2CH_2-$ oder eine einfache Kovalenzbindung bezeichnet und Ring A und $R^1$ die oben gegebenen Bedeutungen haben,

katalytisch hydriert, oder

b) zur Herstellung der Verbindungen der Formel I, worin $X^1$ eine Aethylengruppe, $X^2$ eine einfache Kovalenzbindung und Ring A 1,4-Phenylen bedeuten, eine Verbindung der allgemeinen Formel

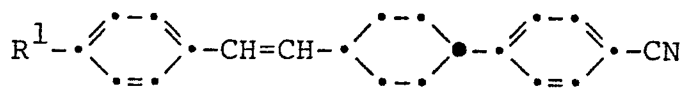 III

worin $R^1$ die oben gegebene Bedeutung hat, katalytisch hydriert, oder

c) zur Herstellung der Verbindungen der Formel I, worin $X^1$ eine Aethylengruppe, $X^2$ eine einfache Kovalenzbindung und Ring A trans-1,4-Cyclohexylen bedeuten, eine Verbindung der allgemeinen Formel

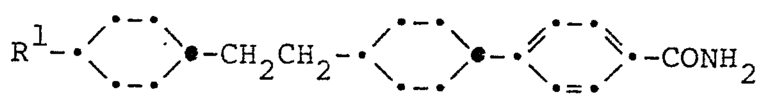 IV

worin $R^1$ die oben gegebene Bedeutung hat, dehydratisiert.

Die katalytische Hydrierung der Verbindungen der Formeln II und III kann in an sich bekannter Weise mit irgendeinem üblichen Hydrierkatalysator, wie Palladium, Platin, Raney-Nickel und dergleichen, vorzugsweise mit Palladium durchgeführt werden. Als Lösungsmittel wird zweckmässig ein unpolares inertes organisches Lösungsmittel verwendet, beispielsweise ein gesättigter Aether oder Ester oder, vorzugsweise, ein gesättigter oder aromatischer Kohlenwasserstoff, wie Hexan, Toluol und dergleichen. Temperatur und Druck sind nicht kritisch. Zweckmässigerweise werden eine Temperatur zwischen Raumtemperatur und der Siedetemperatur des Reaktionsgemisches und ein Druck von etwa 1-5 Atmosphären angewendet.

Die Dehydratisierung eines Amids der Formel IV kann ebenfalls in an sich bekannter Weise erfolgen. Geeignete Dehydratisierungsmittel sind beispielsweise Phosphoroxychlorid, Phosphorpentoxid, Thionylchlorid, Acetanhydrid

und insbesondere Benzolsulfochlorid. Die Dehydratisierung kann gewünschtenfalls in einem inerten organischen Lösungsmittel (z.B. einem Kohlenwasserstoff oder Halogenkohlenwasserstoff) und/oder in Gegenwart einer Base (z.B. Pyridin oder Triäthylamin) durchgeführt werden. Temperatur und Druck sind nicht kritisch. Vorzugsweise wird jedoch bei etwa 50°C bis Rückflusstemperatur und Atmosphärendruck gearbeitet.

Die Verbindungen der Formeln II-IV sind neu und ebenfalls Gegenstand der vorliegenden Erfindung. Sie können in an sich bekannter Weise aus bekannten Verbindungen hergestellt werden.

Beispielsweise können die Verbindungen der Formel II durch Wittig-Reaktion aus p-Cyanobenzyl-triphenylphosphoniumhalogenid und einem geeigneten Cyclohexancarboxaldehyd hergestellt werden. Die Cyclohexancarboxaldehyde sind aus den entsprechenden, bekannten Cyclohexancarbonitrilen durch Reduktion mit Diisobutylaluminiumhydrid erhältlich.

Die Verbindungen der Formel III können beispielsweise durch Wittig-Reaktion aus 4-(p-Cyanophenyl)cyclohexancarboxaldehyd und einem p-(Alkyl oder Alkoxy)benzyl-triphenylphosphoniumhalogenid hergestellt werden. 4-(p-Cyanophenyl)-cyclohexancarboxaldehyd ist aus 4-(p-Cyanophenyl)cyclohexanon durch Umsetzung mit Methoxymethyl-triphenylphosphoniumchlorid und anschliessende Hydrolyse des Enoläthers erhältlich.

Die Verbindungen der Formel IV können beispielsweise dadurch erhalten werden, dass man 2-(trans-4-Alkylcyclohexyl)äthyl-triphenylphosphoniumhalogenid in einer Wittig-Reaktion mit 4-(p-Cyanophenyl)cyclohexanon umsetzt, das erhaltene ungesättigte Nitril verseift und anschliessend in Gegenwart von Palladium katalytisch hydriert, die erhaltene gesättigte Carbonsäure z.B. mit Thionylchlorid in

das Säurechlorid überführt und dieses schliesslich mit Ammoniak umsetzt. Eine weitere Methode zur Herstellung der Verbindungen der Formel IV besteht darin, dass man 2-(trans-4-Alkylcyclohexyl)äthyl-triphenylphosphoniumhalogenid in einer Wittig-Reaktion mit 4-Phenylcyclohexanon umsetzt, die erhaltene Verbindung in Gegenwart von Palladium katalytisch hydriert und durch Friedel-Crafts-Acylierung mit Acetylchlorid in Gegenwart von Aluminiumtrichlorid und anschliessende Umsetzung mit Hypohalogenit (z.B. Natriumhypochlorit) in die Carbonsäure überführt und schliesslich letztere wie oben erwähnt über das Säurechlorid zum gewünschten Amid weiter umsetzt. Die 2-(trans-4-Alkylcyclohexyl)äthyl-triphenylphosphoniumhalogenide können dadurch hergestellt werden, dass man trans-4-Alkylcyclohexancarboxaldehyd mit Methoxymethyl-triphenylphosphoniumchlorid umsetzt, den Enoläther hydrolysiert, den Aldehyd mit Natriumborhydrid reduziert und den erhaltenen Alkohol schliesslich über das Halogenid in das Phosphoniumsalz überführt.

Die erfindungsgemässen flüssigkristallinen Gemische enthalten mindestens 2 Komponenten, wovon mindestens eine Komponente eine Verbindung der Formel I ist. Zusätzlich zu einer oder mehreren Verbindungen der Formel I können die erfindungsgemässen Gemische eine oder mehrere weitere geeignete , flüssigkristalline oder nicht-flüssigkristalline Substanzen enthalten, wie z.B. Substanzen aus den Klassen der Schiffschen Basen, Azobenzole, Azoxybenzole, Ester mit 2-5 carbocyclischen Ringen, Biphenyle, Terphenyle, Phenylcyclohexane, Cyclohexylbiphenyle, Pyrimidine, Dioxane, Bicyclooctane, Aethanderivate mit 2-4 carbocyclischen Ringen und dergleichen. Derartige Substanzen sind dem Fachmann bekannt und viele davon sind zudem im Handel erhältlich.

Vorzugsweise enthalten die erfindungsgemässen Flüssigkristallmischungen eine oder mehrere Verbindungen der Formel I und eine oder mehrere der Verbindungen der folgenden

allgemeinen Formeln

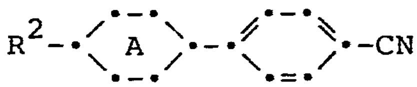

V

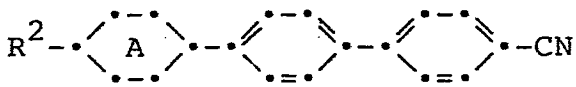

VI

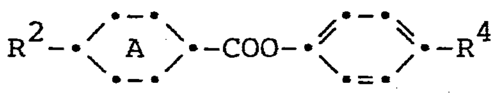

VII

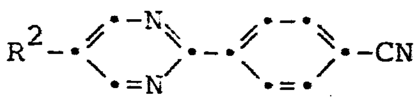

VIII

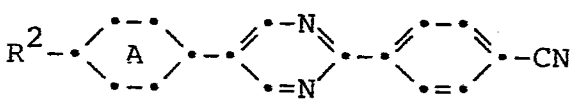

IX

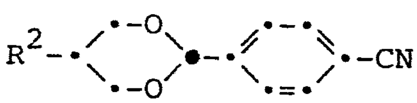

X

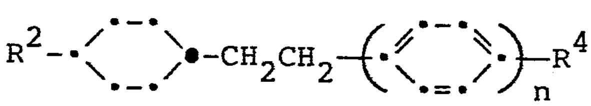

XI

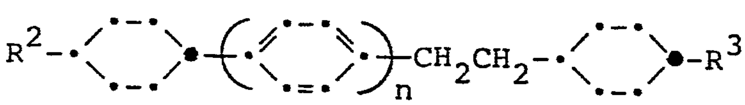

XII

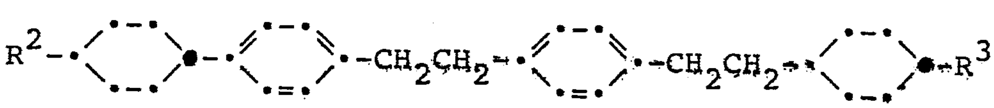

XIII

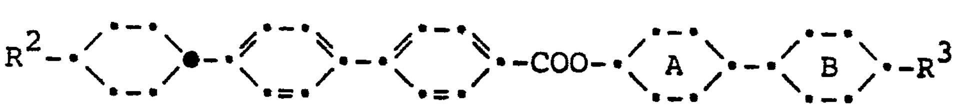

XIV

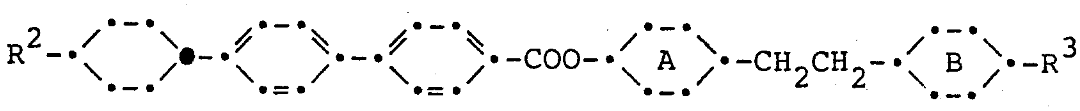 XV

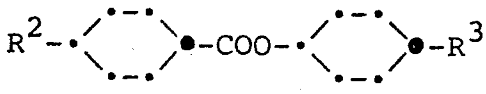 XVI

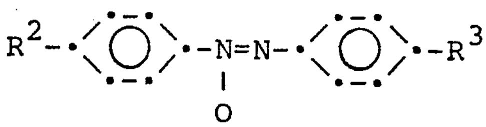 XVII

worin $R^2$ und $R^3$ geradkettiges $C_1$-$C_7$-Alkyl bedeuten, $R^4$ Cyano, geradkettiges $C_1$-$C_7$-Alkyl oder geradkettiges $C_1$-$C_7$-Alkoxy bezeichnet, n für die Zahl 1 oder 2 steht und die Ringe A und B 1,4-Phenylen oder trans-1,4-Cyclohexylen darstellen.

Die erfindungsgemässen Mischungen können ferner geeignete optisch aktive Verbindungen (z.B. optisch aktive Biphenyle) und/oder dichroitische Farbstoffe (z.B. Azo-, Azoxy- und Anthrachinon-Farbstoffe) enthalten. Der Anteil solcher Verbindungen wird durch die Löslichkeit und die gewünschte Ganzhöhe (pitch), Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gew.-%.

Der Anteil an Verbindungen der Formel I in den erfindungsgemässen Mischungen kann in breiten Grenzen variieren. Die erfindungsgemässen Mischungen können auch aus 2 oder mehreren Verbindungen der Formel I bestehen. Im allgemeinen enthalten sie jedoch mindestens etwa 1 Gew.-% und höchstens etwa 25 Gew.-% an Verbindungen der Formel I. Vorzugsweise beträgt der Anteil an Verbindungen der Formel I etwa 3-15 Gew.-%.

Die Herstellung der flüssigkristallinen Gemische und der elektro-optischen Vorrichtungen kann in an sich bekannter Weise erfolgen.

Die Verwendung der erfindungsgemässen Verbindungen für oder als flüssigkristalline stationäre Phasen in der Gaschromatographie kann in an sich bekannter Weise und auf üblichen Trägermaterialien erfolgen. Besonders geeignet sind die erfindungsgemässen Verbindungen zur Trennung von Isomerengemischen, welche mit konventionellen stationären Phasen nicht oder nur schlecht trennbar sind.

Die Erfindung betrifft ferner alle neuen Verbindungen, Mischungen, Verfahren, Verwendungen und Vorrichtungen wie hierin beschrieben.

Die Herstellung der erfindungsgemässen Verbindungen wird anhand der nachstehenden Beispiele weiter veranschaulicht. C bezeichnet eine kristalline, S eine smektische, N eine nematische und I die isotrope Phase.

Beispiel 1

6,0 g p-[2-[trans-4-[2-(trans-4-Propylcyclohexyl)-äthyl]cyclohexyl]vinyl]benzonitril wurden in 400 ml Hexan gelöst und mit 0,8 g Palladium/Kohle (10%) bei Raumtemperatur und Normaldruck bis zum Stillstand der Wasserstoffaufnahme hydriert. Nach dem Abfiltrieren des Katalysators wurde das p-[2-[trans-4-[2-(trans-4-Propylcyclohexyl)-äthyl]cyclohexyl]äthyl]benzonitril zweimal aus Hexan umkristallisiert; Smp. (C-N) 91,5°C, Klp. (N-I) 167°C.

Das als Ausgangsmaterial verwendete p-[2-[trans-4-[2-(trans-4-Propylcyclohexyl)äthyl]cyclohexyl]vinyl]benzonitril wurde wie folgt hergestellt:

a) Bei 0°C und unter Inertgasatmosphäre wurden zu einer Lösung von 5,5 g trans-4-[2-(trans-4-Propylcyclohexyl)-äthyl]cyclohexancarbonitril in 500 ml Toluol innert 30 Minuten 24 ml einer 1,2M Lösung von Diisobutylaluminiumhydrid in Toluol zugetropft und das Reaktionsgemisch noch 1 Stunde bei 0°C und 3 Stunden bei Raumtemperatur gerührt. Anschliessend wurde das Reaktionsgemisch auf 500 ml 0,5N Schwefelsäure gegossen und dreimal mit je 200 ml Diäthyläther extrahiert. Die vereinigten organischen Phasen wurden zuerst mit gesättigter Natriumhydrogencarbonat-Lösung und dann mit Wasser gewaschen, über Natriumsulfat getrocknet und eingeengt, wobei 5,6 g trans-4-[2-(trans-4-Propylcyclohexyl)äthyl]cyclohexancarboxaldehyd erhalten wurden.

b) Ein Gemisch von 5,6 g trans-4-[2-(trans-4-Propylcyclohexyl)äthyl]cyclohexancarboxaldehyd, 10,6 g p-Cyanobenzyltriphenylphosphoniumbromid und 200 ml Dioxan wurde mit 14,5 g pulverisiertem Kaliumcarbonat versetzt und 72 Stunden zum Sieden erhitzt. Nach dem Erkalten wurde das Reaktionsgemisch filtriert, das Filtrat eingeengt und der Rückstandstand an Kieselgel mit Hexan/Diäthyläther (Vol. 4:1) chromatographiert, wobei 6,0 g p-[2-[trans-4-[2-(trans-4-Propylcyclohexyl)äthyl]cyclohexyl]vinyl]benzonitril erhalten wurden.

Beispiel 2

7,0 g p-[4-[2-(p-Pentylphenyl)vinyl]cyclohexyl]benzonitril wurden in 350 ml Toluol gelöst, mit 1,4 g Palladium/Kohle (10%) versetzt und bei Raumtemperatur und Normaldruck bis zum Stillstand der Wasserstoffaufnahme hydriert. Das nach dem Abfiltrieren des Katalysators und dem Einengen des Filtrats erhaltene, rohe p-[trans-4-[2-(p-Pentylphenyl)äthyl]cyclohexyl]benzonitril wurde zweimal aus Aethanol umkristallisiert. Ausbeute 4,2 g; Smp. (C-N) 61,5°C, Klp. (N-I) 126°C.

Das als Ausgangsmaterial verwendete p-[4-[2-(p-Pentylphenyl)vinyl]cyclohexyl]benzonitril wurde wie folgt hergestellt:

a) Unter Inertgasatmosphäre und Rühren wurde eine Suspension von 27,9 g Methoxymethyl-triphenylphosphoniumchlorid in 200 ml trockenem t-Butylmethyläther bei -10°C innert 10 Minuten portionenweise mit 9,5 g Kalium-t-butylat versetzt. Nach weiteren 30 Minuten wurde zum tieforangen Gemisch bei 0-5°C eine Lösung von 10,8 g 4-(p-Cyanophenyl)-cyclohexanon in 125 ml trockenem Tetrahydrofuran zugetropft. Das Reaktionsgemisch wurde noch 30 Minuten bei 0°C und 2,5 Stunden bei Raumtemperatur gerührt, dann auf 1200 ml Hexan gegossen und filtriert. Das Filtrat wurde eingeengt und der Rückstand an Kieselgel mit Hexan/Aethylacetat (Vol. 95:5) chromatographiert. Die gemäss Dünnschichtchromatographie reinen Fraktionen von p-[4-(Methoxymethyliden)cyclohexyl]benzonitril wurden eingeengt, wobei 11,0 g farbloses Oel erhalten wurden.

b) Das erhaltene Oel (11,0 g) von p-[4-(Methoxymethyliden)-cyclohexyl]benzonitril wurde in einem Gemisch von 200 ml Tetrahydrofuran und 50 ml 2N Salzsäure 1 Stunde zum Sieden erhitzt. Anschliessend wurde das Reaktionsgemisch auf

 

500 ml Eiswasser gegossen und dreimal mit je 200 ml Diäthyläther extrahiert. Die organischen Phasen wurden mit Wasser neutral gewaschen, über Natriumsulfat getrocknet und eingeengt. Hierbei wurden 10,0 g farbloser, zum Teil fester 4-(p-Cyanophenyl)cyclohexancarboxaldehyd (cis/trans-Verhältnis ca. 1:3) erhalten.

c) Ein Gemisch von 4,6 g des erhaltenen 4-(p-Cyanophenyl)-cyclohexancarboxaldehyds, 15 g pulverisiertem Kaliumcarbonat, 16,2 g p-Pentylbenzyl-triphenylphosphoniumbromid und 300 ml Dioxan wurde 72 Stunden zum Sieden erhitzt. Nach dem Erkalten wurde das Reaktionsgemisch filtriert und das Filtrat eingeengt. Der Rückstand wurde in 400 ml Hexan aufgeschlämmt und durch Filtration vom ungelösten Triphenylphosphinoxid befreit. Das Filtrat wurde eingeengt und das erhaltene Rohprodukt durch Chromatographie an Kieselgel mit Hexan/Aethylacetat (Vol. 95:5) gereinigt. Hierbei wurden 7,0 g p-[4-[2-(p-Pentylphenyl)vinyl]cyclohexyl]benzonitril erhalten (cis/trans-Verhältnis am Cyclohexanring ca. 15:85, E/Z-Verhältnis ca. 85:15).

In analoger Weise wurde folgende Verbindung hergestellt, wobei anstelle von 4-(p-Cyanophenyl)cyclohexanon 4-(p-Pentylphenyl)cyclohexanon und anstelle p-Pentylbenzyl-triphenylphosphoniumbromid p-Cyanobenzyl-triphenylphosphoniumbromid eingesetzt wurde:

p-[2-[trans-4-(p-Pentylphenyl)cyclohexyl]äthyl]benzonitril; Smp. (C-N) 77,5°C, Klp. (N-I) 120°C.

## Beispiel 3

Zu einer Suspension von 2,6 g rohem p-[trans-4-[2-(trans-4-Pentylcyclohexyl)äthyl]cyclohexyl]benzamid in 50ml Pyridin wurden 1,85 g Benzolsulfochlorid zugegeben und das Gemisch über Nacht bei 55°C gerührt. Die erhaltene Lösung wurde auf Wasser ausgegossen und das Produkt mit Diäthyläther extrahiert. Der Aetherextrakt wurde viermal mit je 50 ml 3N Salzsäure, einmal mit 50 ml gesättigter

Natriumhydrogencarbonat-Lösung und dreimal mit je 100 ml Wasser gewaschen, über Natriumsulfat getrocknet und eingeengt. Das rohe p-[trans-4-[2-(trans-4-Pentylcyclohexyl)-äthyl]cyclohexyl]benzonitril (2,4 g) wurde dreimal aus Hexan umkristallisiert; Ausbeute 1,1 g; Smp. (C-N) 61,5°C bzw. 67°C (2 Modifikationen), Klp. (N-I) 193°C.

Das als Ausgangsmaterial verwendete p-[trans-4-[2-(trans-4-Pentylcyclohexyl)äthyl]cyclohexyl]benzamid wurde wie folgt hergestellt:

a) In analoger Weise zu Beispiel 2 Absätze a) und b) wurde trans-4-Pentylcyclohexancarboxaldehyd in einer Wittig-Reaktion mit Methoxymethyl-triphenylphosphoniumchlorid und Kalium-t-butylat umgesetzt und anschliessend der entstandene Enoläther in Tetrahydrofuran/2N Salzsäure zum (trans-4-Pentylcyclohexyl)acetaldehyd hydrolysiert.

b) Ein Gemisch von 35,1 g (trans-4-Pentylcyclohexyl)-acetaldehyd und 400 ml Aethanol/Wasser (Vol. 1:1) wurde portionenweise mit 36 g Natriumborhydrid versetzt und noch 1 Stunde gerührt. Dann wurde das Reaktionsgemisch auf 1200 ml Wasser gegossen und dreimal mit je 400 ml Diäthyläther extrahiert. Die organischen Phasen wurden mit Wasser neutral gewaschen, über Natriumsulfat getrocknet und eingeengt. Ausbeute: 34,2 g 2-(trans-4-Pentylcyclohexyl)äthanol als gelbliches Oel.

c) Unter Inertgasatmosphäre und Rühren wurde ein Gemisch von 20 g 2-(trans-4-Pentylcyclohexyl)äthanol, 27,5 g Triphenylphosphin und 350 ml Dichlormethan bei -30°C innert 45 Minuten portionenweise mit 36,5 g Tetrabrommethan versetzt und dann noch 30 Minuten bei -20°C und 5 Stunden bei Raumtemperatur gerührt. Anschliessend wurde das Reaktionsgemisch eingeengt und der Rückstand mit 500 ml Hexan trituriert. Nach Abkühlen auf 0°C wurde das ausgefallene Triphenylphosphinoxid abfiltriert und das Filtrat an Kieselgel mit Hexan chromatographiert. Das erhaltene, rohe 1-Brom-2-(trans-4-pentylcyclohexyl)äthan (29,0 g) wurde bei 92-98°C/ 1,5 Torr destilliert. Ausbeute: 21,9 g als farbloses Oel.

d) Ein Gemisch von 13,0 g 1-Brom-2-(trans-4-pentylcyclohexyl)äthan, 16,0 g Triphenylphosphin und 375 ml Xylol wurde 60 Stunden zum Sieden erhitzt. Nach Abkühlen auf 5°C wurde das ausgefallene Produkt abgenutscht, mit Toluol und Hexan gewaschen und getrocknet, wobei 15,0 g 2-(trans-4-Pentylcyclohexyl)äthyl-triphenylphosphoniumbromid erhalten wurden; Smp. 183-185°C.

e) Eine Suspension von 10,6 g 2-(trans-4-Pentylcyclohexyl)äthyl-triphenylphosphoniumbromid in 100 ml trockenem t-Butylmethyläther wurde unter Inertgasatmosphäre bei 5-10°C portionenweise mit 2,2 g Kalium-t-butylat versetzt und noch 10 Minuten gerührt. Anschliessend wurde innert 30 Minuten eine Lösung von 3,5 g 4-(p-Cyanophenyl)cyclohexanon in 50 ml Tetrahydrofuran zur orangen Suspension zugetropft. Das Reaktionsgemisch wurde über Nacht bei Raumtemperatur gerührt und dann mit 50 ml Wasser versetzt. Die wässrige Phase wurde abgetrennt und mit Diäthyläther extrahiert. Die vereinigte organische Phase wurde mit Wasser neutral gewaschen, über Natriumsulfat getrocknet und eingeengt. Der Rückstand wurde an Kieselgel mit Hexan/Aethylacetat (Vol. 95:5) chromatographiert, wobei 3,8 g p-[4-[2-(trans-4-Pentylcyclohexyl)äthyliden]cyclohexyl]-benzonitril erhalten wurden.

f) Ein Gemisch von 3,5 g rohem p-[4-[2-(trans-4-Pentylcyclohexyl)äthyliden]cyclohexyl]benzonitril und 100 ml Aethylenglykol wurde mit 2,0 g Kaliumhydroxid versetzt und dann 7 Stunden bei einer Badtemperatur von 190°C zu leichtem Sieden erhitzt. Nach dem Erkalten wurde das Reaktionsgemisch auf Wasser gegossen und mit 3N Salzsäure angesäuert. Das ausgefallene Produkt wurde in Diäthyläther aufgenommen. Der Aetherextrakt wurde dreimal mit je 100 ml Wasser gewaschen, über Natriumsulfat getrocknet und eingeengt. Die erhaltene rohe p-[4-[2-(trans-4-Pentylcyclo-

hexyl)äthyliden]cyclohexyl]benzoesäure (3,6 g) wurde in 500 ml Aethanol und 125 ml Toluol gelöst und mit 0,6 g Palladium/Kohle (10%) 48 Stunden bei Raumtemperatur und Normaldruck hydriert. Der Katalysator wurde abfiltriert und das Filtrat eingeengt, wobei 3,1 g rohe, feste p-[trans-4-[2-(trans-4-Pentylcyclohexyl)äthyl]cyclohexyl]benzoesäure erhalten wurden.

g) Ein Gemisch von 3,1 g roher p-[trans-4-[2-(trans-4-Pentylcyclohexyl)äthyl]cyclohexyl]benzoesäure, 50 ml Benzol und 0,9 ml Thionylchlorid wurde 2 Stunden zum Sieden erhitzt. Benzol und überschüssiges Thionylchlorid wurden im Vakuum abdestilliert. Der Rückstand wurde zweimal in 50 ml Toluol aufgenommen und eingeengt. Das erhaltene rohe Säurechlorid (3,5 g) wurde in 50 ml Dioxan gelöst und die Lösung in ein Gemisch von 18 ml konzentriertem Ammoniak und 75 ml Dioxan einlaufen gelassen. Die entstandene Suspension wurde 1 Stunde bei Raumtemperatur gerührt, dann mit Wasser verdünnt und genutscht. Das Nutschgut wurde mit Wasser neutral gewaschen und dann durch Einengen mit Toluol getrocknet. Es wurden 2,6 g rohes p-[trans-4-[2-(trans-4-Pentylcyclohexyl)äthyl]cyclohexyl]benzamid erhalten.

In analoger Weise wurde folgende Verbindung hergestellt:

p-[trans-4-[2-(trans-4-Propylcyclohexyl)äthyl]cyclohexyl]benzonitril; Smp. (C-N) 85,7°C, Klp. (N-I) 197,5°C.

0129177

## Patentansprüche

1. Verbindungen der allgemeinen Formel

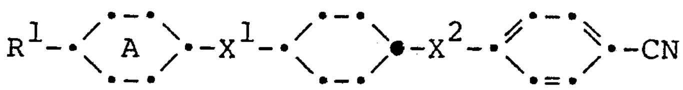

I

worin eine der Gruppen $X^1$ und $X^2$ eine Aethylengruppe $-CH_2CH_2-$ und die andere eine Aethylengruppe $-CH_2CH_2-$ oder eine einfache Kovalenzbindung bezeichnet, Ring A 1,4-Phenylen oder trans-1,4-Cyclohexylen darstellt und $R^1$ geradkettiges $C_1$-$C_{12}$-Alkyl oder an einem 1,4-Phenylenring auch geradkettiges $C_1$-$C_{12}$-Alkoxy bedeutet.

2. Verbindungen nach Anspruch 1, dadurch gekennzeichnet, dass $X^1$ eine Aethylengruppe $-CH_2CH_2-$ und $X^2$ eine einfache Kovalenzbindung bedeuten.

3. Verbindungen nach Anspruch 1, dadurch gekennzeichnet, dass $X^1$ und $X^2$ je eine Aethylengruppe $-CH_2CH_2-$ bedeuten.

4. Verbindungen nach einem der Ansprüche 1 bis 3, dadurch gekennzeichnet, dass $R^1$ geradkettiges $C_1$-$C_7$-Alkyl oder an einem 1,4-Phenylenring auch geradkettiges $C_1$-$C_6$-Alkoxy bedeutet.

5. Verbindungen nach einem der Ansprüche 1 bis 4, dadurch gekennzeichnet, dass $R^1$ geradkettiges Alkyl bedeutet.

6. Flüssigkristallines Gemisch mit mindestens 2 Komponenten, dadurch gekennzeichnet, dass mindestens eine Komponente eine Verbindung der in Anspruch 1 definierten Formel I ist.

7. Verfahren zur Herstellung der Verbindungen der in Anspruch 1 definierten Formel I, dadurch gekennzeichnet, dass man

a) zur Herstellung der Verbindungen der Formel I, worin $X^2$ eine Aethylengruppe bedeutet, eine Verbindung der allgemeinen Formel

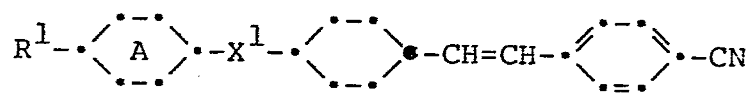

II

worin $X^1$ eine Aethylengruppe $-CH_2CH_2-$ oder eine einfache Kovalenzbindung bezeichnet und Ring A und $R^1$ die in Anspruch 1 gegebenen Bedeutungen haben, katalytisch hydriert, oder

b) zur Herstellung der Verbindungen der Formel I, worin $X^1$ eine Aethylengruppe, $X^2$ eine einfache Kovalenzbindung und Ring A 1,4-Phenylen bedeuten, eine Verbindung der allgemeinen Formel

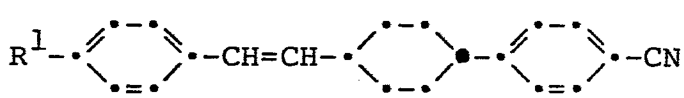

III

worin $R^1$ die in Anspruch 1 gegebene Bedeutung hat, katalytisch hydriert, oder

c) zur Herstellung der Verbindungen der Formel I, worin $X^1$ eine Aethylengruppe, $X^2$ eine einfache Kovalenzbindung und Ring A trans-1,4-Cyclohexylen bedeuten, eine Verbindung der allgemeinen Formel

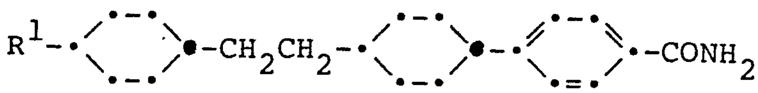

IV

worin $R^1$ die in Anspruch 1 gegebene Bedeutung hat, dehydratisiert.

8. Verwendung der Verbindungen der in Anspruch 1 definierten Formel I für elektro-optische Zwecke.

***